**Europäisches Patentamt**

**European Patent Office**

**Office européen des brevets**

(19)

(11) Publication number: **0 218 759 B1**

# EUROPEAN PATENT SPECIFICATION

(12)

(45) Date of publication of the patent specification:
**24.05.89**

(51) Int. Cl.⁴: **A61B 17/36**

(21) Application number: **85306418.6**

(22) Date of filing: **10.09.85**

(54) Moxibustion method and device.

(43) Date of publication of application:
**22.04.87 Bulletin 87/17**

(45) Publication of the grant of the patent:
**24.05.89 Bulletin 89/21**

(84) Designated Contracting States:
**DE FR GB**

(56) References cited:
**CH-A- 366 127**
**DE-A- 3 118 232**
**US-A- 3 946 733**
**US-A- 4 090 517**
**US-A- 4 325 371**

(73) Proprietor: **Kuratomi, Yasuro, 8-2, 3-chome Kita-Shinjuku, Shinjuku-ku Tokyo(JP)**
Proprietor: **Oshitani, Sei, 1013 Ooaza-Nose Asai-machi, Higashi-asaigun Shiga-ken(JP)**

(72) Inventor: **Kuratomi, Yasuro, 8-2, 3-chome Kita-Shinjuku, Shinjuku-ku Tokyo(JP)**
Inventor: **Oshitani, Sei, 1013 Ooaza-Nose Asai-machi, Higashi-asaigun Shiga-ken(JP)**

(74) Representative: **Kearney, Kevin David Nicholas et al, KILBURN & STRODE 30 John Street, London, WC1N 2DD(GB)**

## Description

The present invention relates to a method and apparatus for carrying out moxibustion treatment, namely the burning of the moxa herb in close proximity to or contact with the skin.

Moxibustion treatment involving such application of moxa has long been used in oriental medicine. It manifests significant curative effects on pain of the nerve system of the body including the motor nerve, the autonomic nerve, sensory nerves or on pain due to psychoneurosis Moxibustion has special advantages in that moxa contains many ingredients including adenine, choline and highly volatile thymol. Thus, when the moxa is burnt the above ingredients are vapourized and are readily absorbed through the skin into the tissue of the body or blood, and effectively act on the cells of the human body. A further specific feature is that of improving functions by the stimulation of heat.

In a conventional moxibustion treatment, moxa is placed on a certain key point, and the moxa ignited or burnt. This method is considered to have the merit that skin tissue is burnt, and a part of the dead tissue is absorbed from the skin into the blood vessels and is though to make various immunizing materials in the blood vessels. However, it has the disadvantage that it causes severe pain at and near the point of application, leaves a scar from the burn on the skin and generates a burning smell. In addition it is necessary for the treatment to be performed by a specialist in moxibustion treatment.

In order to avoid leaving a scar a modified process known as "no-scar moxibustion" became to be used. This method involves placing a vegetable or herb material, e.g. ginger, garlic, onion, leek, or miso, on the skin, placing the moxa on such material and igniting the moxa. The procedure does not make one feel so hot, and leaves no scar. However, since this method only utilizes warm or lower heat temperature it is not thought to utilize the ingredients of moxa fully.

The present invention aims to achieve an effective moxibustion treatment whereby the ingredients of moxa are applied to the body to achieve curative effects without the use of fire. The procedure of the present invention is such as to full vapourize the ingredients of the moxa without using fire, whilst fully exhibiting the effect of heating though not leaving scarring.

The device of the present invention feeds air to a pyrogen provided by a heat generating composition which generates heat by oxidation. Herb material composed of moxa or a mixture of moxa and other medicinal herbs is located in contact with the pyrogen, the heat from which vapourizes the ingredients of the moxa and medicinal herbs, and the vapour or fumes and heat so produced act on the surface of the skin on which the device is positioned.

The pyrogen to be used in the present invention involves a heat generating composition such as iron powder, carbon, cellulose, chlorides, and water.

The powdered iron is mainly used in an amount of 42 wt%-51 wt% preferably in the form of iron-oxide. The carbon is mainly in an amount of 18 wt%-22 wt% e.g. as active carbon. The cellulose is mainly used in an amount of 15.5 wt%-19 wt% e.g. as wood wool (excelsior). The chloride is mainly used in an amount of 3.7 wt%-4.5 wt% e.g. as sodium chloride. The water content is desirably 11 wt%-13.5 wt%.

When oxygen, e.g. as air, is supplied to such a heat generating composition, heat will be generated by oxidation of the iron-oxide.

Since the pyrogen used in the present invention generates heat by reduction with oxygen in the air, it must be stored out of contact with air. For this purpose, a material impermeable to oxygen should be used to pack and store the pyrogen out of contact with air and desirably means should be provided to enable air to get access to the pyrogen when the device is to be used.

However within this external oxygen impermeable package the pyrogen is desirably housed in a gas permeable package. Thus the pyrogen may be accommodated in an internal package which may be formed of woven fabric e.g. of synthetic resin or cotton, gas permeability being provided by the gaps between fibres, or a non-woven fabric e.g. of synthetic resin, cotton, or a film provided with an appropriate number of ventilation holes. The exterior of the internal package is covered by the external package formed of non-permeable material e.g. of synthetic resin film or thin sheet, which keeps the pyrogen out of contact with air. The external package may be arranged to be torn off at the time of application of the device to the skin to enable oxygen in the air to contact the pyrogen and start the heat generating process.

The structure can also be one where the non-permeable envelope (e.g. formed of synthetic resin film, thin sheet or unwoven fabric) will be perforated at the time of use. Another arrangement which may be used is where the external non-permeable envelope is prepared with ventilation holes which are sealed during storage of the device prior to use by a sealing plate or strip, which is peeled off at the time of application to open the ventilation hole or holes.

The pyrogen as described above can be adjusted to give the desired heat generation time and temperature as desired by adjusting the composition of the material or the ventilating structure. However, in normal cases 10g-20g of the pyrogen is sufficient, 10g of pyrogen typically affording continuous heat generation for a time of 3-4 hrs and 50g of pyrogen affording continuous heat generation for 5 hrs-6 hrs. The temperature at the heat generation source is of the order of 65°C whilst the temperature at the point of contacting with the skin is about 40°C-45°C.

As mentioned above the moxa can be used on its own as the sole herb material or can be mixed with other vegetable or herb materials e.g. ginger, garlic, Swertia japoneca, or Angelica utilis, to increase the effect further and in such case the amount of such other vegetable or herb material is preferably 1g-3g.

The herb material can be filled into the package as is, however, it is more convenient to use a herb supporting board preferably made of flexible material e.g. synthetic unwoven fabric, paper or cloth, desirable coated on one side with adhesive, the herb material being adhered to the adhesive by being pressed onto it. The herb material must be arranged so as to be in contact with the pyrogen to ensure that the ingredients of the herb composition are vapourised when the pyrogen generates heat and can then act on the skin. Thus, the herb material is packed in the package in such a way that it contacts the bag containing the pyrogen, or the above herb supporting board with the adhered herb material is placed in contact with the pyrogen containing bag.

The skin side of the package body is preferably constructed so as to be open, and this opening may be completely open packed so that gas permeability will be maintained, or covered e.g. pasted with a separate piece, e.g. a sheet, having gas permeability. The heat generated by the pyrogen and the vapours generated from the herb material come into contact with the skin surface through this opening and the effects of moxibustion can be expected.

In addition, the face of the package body which is to be applied to the skin may be formed to allow it to be moved at will from place to place during use, or may be provided with adhesive so that it will be adhered to the skin surface at the predetermined key point.

The device of the present invention heats the herb material by the heat of the oxidation of the pyrogen, the ingredients of the herb material are vapourized by this heat, and this generates the required vapours. Infrared rays and heat are radiated by these vapours and heat thus acts on the skin surface, and activates the functions of the cells.

This arrangement eliminates the dangers of using fire or burning of the moxa as in the conventional method. The method is thus simple and easy, and is safe, it does not leave a scar on the skin, no burning smell is generated, and also the functions of the cells are activated by injection of infrared rays.

One preferred form of a composition of pyrogen for use in the present invention e.g. for a 20 gram amount of pyrogen is as follows.

| Iron oxide | 90 mesh | 46.4 wt% |
|---|---|---|
| Active carbon | 100 mesh | 20 wt% |
| Excelsior wood wool | | 17.2 wt% |
| Sodium chloride | | 4.1 wt% |
| Water | | 12.3 wt% |

A preferred form of device in accordance with the present invention has the above pyrogen filled into an internal package body made of unwoven fabric of synthetic resin, many small ventilation holes being formed in the internal package body. A paper support with 2g of moxa on its own adhered to it is located on or in the internal package so that the paper contacts the internal package, the moxa side of the paper being held by adhering gas-permeable woven cloth around the internal package, and the whole is wrapped in non-permeable external package body. In use the external package is torn off prior to application of the device to the skin. When this is done and the internal package applied to the skin the pyrogen will typically generate heat for 5 hrs-6 hrs, and the vapours generated from the moxa permeate to the surface of the skin to which the device is applied.

The invention may be put into practice in various ways and a number of specific embodiments will be described to illustrate the invention with reference to the accompanying example and drawings, in which:

Figure 1 is a perspective view of a first embodiment of a device in accordance with the present invention;

Figure 2 is a longitudinal sectional view of the device of Figure 1;

Figure 3 is a sectional view of a second embodiment of a device in accordance with the present invention;

Figure 4 is a perspective view of a third embodiment of a device in accordance with the present invention;

Figure 5 is a perspective view of a herb supporting plate for use in the third embodiment;

Figure 6 is a perspective view of a package body attached to a herb supporting plate for use in the third embodiment;

Figure 7 is a perspective view of an adhesion plate for use in the third embodiment; and

Figure 8 is a perspective view indicating the whole assembly of the third embodiment with the sealing strip being peeled away prior to application to the skin and activation of the pyrogen.

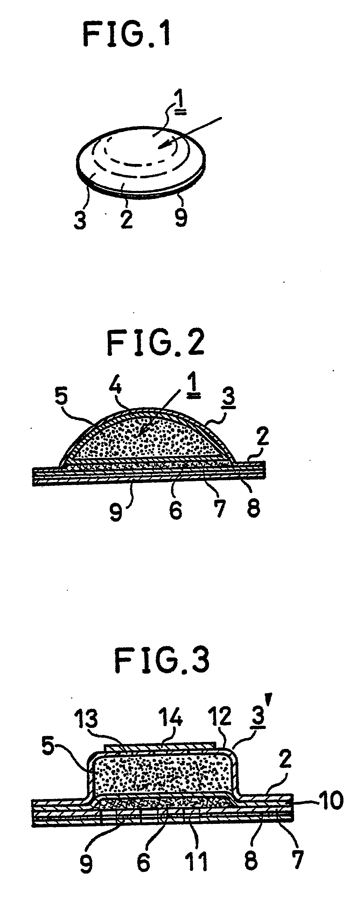

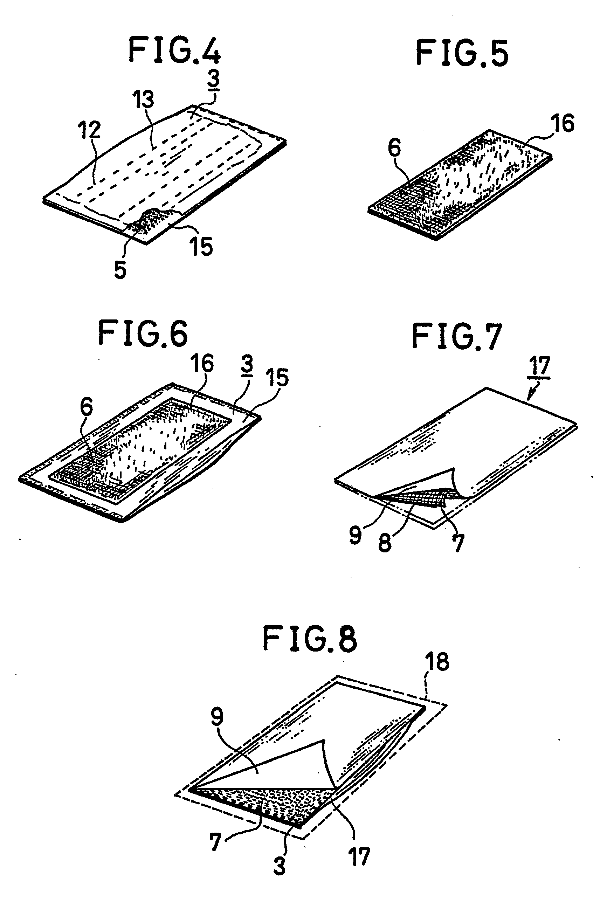

Referring now to Figures 1 and 2 a first embodiment of a moxibustion apparatus or device 1 is shown which has a central circular protruberance. This circular saucer type package body 3 is made of non-permeable material such as paper, synthetic paper, synthetic resin, and has a flange shaped rim 2. A supporting body 7 of good gas-permeability is adhered to the bottom side of the rim 2 and carries the herb material 6. A adhesive agent 8 is applied to the under surface of the supporting body 7, and the adhesive agent 8 is pasted with a non-gas-permeable peelable sealing paper or strip 9. A pyrogen 5 is located inside the package so formed inside the protruberance. The pyrogen is enclosed within a non-permeable bag body 4 made of synthetic resin film.

A layer of herb material 6, which is moxa on its own or a mixture of moxa and other herbs, is pressed into tight contact with the underside of the bag body 4. This herb material 6 may alternatively be packed in paper or cloth of good gas-permeability and accommodated in the package body 3.

In use, one first peels off the sealing paper 9, and forms a ventilating hole through the outer side of the top wall of the package body 3 to expose the pyrogen 5 to air. The device is adhered to the skin surface corresponding to the key point of the human body by the adhesive agent 8. Air then intrudes through the said ventilation hole or holes, and the pyrogen 5 starts to be oxidized by the oxygen in the air and generates heat. This heat generation will cause the ingredients of the herb material 6 to vapourize, and these vapours, heat produced by the heat generation and infrared rays caused by the heat will act on the surface of the skin through the supporting body 7, and the moxibustion effect can be expected to occur.

Referring now to Figure 3 a second embodiment will be described. A central part affords a circular protruberance 3' within which the pyrogen 5 is accommodated. The package body 3' is formed of non-permeable material such as synthetic resin. The package body again has a flange shaped rim 2, and a dished paper partition plate 10 is adhered to the under side of the rim 2 to support the pyrogen 5. The herb material 6 is placed in the dished centre of the under side of the partition plate 10 so that the herb material forms a layer. A supporting body 7 is adhered to the periphery of the under side of the partition plate 10 so as to support the herb material 6 to hold it close against the partition plate 10. This supporting body 7 is made of a thin film of synthetic resin with many ventilation holes 11. The under side of the supporting body 7 is provided with a layer of adhesive agent 8, and a peelable sealing paper or strip 9 is pasted to the adhesive agent 8. The air ventilation holes 11 extend through the supporting body 7, the layer of adhesive agent 8 and the peelable sealing paper or strip. Vapourised herb material may reach the skin through the ventilation holes 11.

A number of air feeding holes 13 are formed in the top wall 12 of the package body 3, and a peelable sealing plate or strip 14 is pasted to the upper surface cf the top wall 12, closing the air feeding holes 13, and preventing intrusion of air into the package body 3.

In use, the sealing plate 14 will be peeled off, the air feeding holes 13 thus being opened. The peeling paper 9 is peeled off and the device is secured to the skin corresponding to the key point of the human body by the adhesive 8. Oxidation of the pyrogen 5 generates heat and vapours from the herb material 6 as in the first embodiment.

This second embodiment is very convenient in that air is fed to the pyrogen 5 by removing the sealing plate 14 rather than having to form holes in the wall 3'.

The third embodiment will now be described with reference to Figures 4 to 8. The package body 3 has a non-gas-permeable rectangular top wall 12 and bottom wall 15 which are formed of synthetic resin unwoven fabric. The pyrogen 5 located between the top and bottom walls, and the circumference of the walls 12 and 15 is heat sealed to seal in the pyrogen 5. Air feeding holes 13 consisting of many small holes are formed in the top wall 12.

A herb supporting plate 16 (see Figure 5) consists of e.g. paper, unwoven fabric or gauze, and of rectangular shape, carries herb material 6 consisting of moxa on its own or moxa mixed with other herbs adhered to the plate 16 by adhesive agent or electrostatic adhesion. The herb supporting plate 16 formed in this way is adhered to or loaded to the centre of the bottom wall 15, so that the herb material 6 is located against the under side of the wall 15.

An adhesion plate 17 is formed by a supporting plate 7 formed of gas-permeable cloth e.g. of unwoven fabric, having elasticity and wettability and coated with adhesive agent 8, and covered with a peelable sealing paper or strip 9 as shown in Figure 7. The supporting body 7 is adhered, e.g. by heat sealing, to the circumferential rim and penetrating adhesive agent 8 to the circumferential rim of the bottom wall 15 so that the body 7 will cover the herb supporting plate 16.

The package body 3 constituted as above and with the adhesion plate 17 adhered to it is packed within a non-gas-permeable external package bag 18 as shown by chain lines in Figure 8 to form a moxibustion device or apparatus in accordance with the present invention.

In use the external package bag 18 will first be torn off. This results in air entering through the air feeding holes 13 of the package body 3, and the pyrogen 5 will generate heat on oxidation by the oxygen in the air. The peeling paper 9 located on the one side of the package body 3 is then peeled off, and the device is applied to the skin area corresponding to the key point or affected part of the human body with the adhesive agent 8 attached to the supporting body 7.

Warm heat produced by the heat generation of the pyrogen 5, is transmitted to the herb material 6 through the package body 3, and vapours of the ingredients are generated by the warm heat, and these vapours permeate from the supporting body 7 through the skin, and perform the moxibustion treatment.

Because the herb material 6 contacts the skin through the supporting body 7 which has elasticity and wettability, the supporting body 7 affords an air layer, which absorbs sweat and transfers heat to the body from the skin, performing the function of a thermal cushion, and successively releases accumulated heat in the human body to outside of the body, and is able to maintain the body temperature at the proper temperature in the range 40°C-45°C.

Air feeding holes 13 are located in the top wall of the package body 3, however, the supporting body 7 need not be adhered for over the whole of its area with the skin surface, and thus additional air feeding holes 13 may be made in the bottom wall 15, so that the air can intrude through the supporting body 7 to also supply oxygen to the pyrogen 5. Thus both top and bottom walls 12 and 15 may be prepared with air feeding holes or one or other of them.

Culpak processed Kraft paper may be used as the herb supporting plate 16 for adhering the herb material 6, and its black colour provides good heat conduction and results in the whole surface of the herb supporting plate 16 being heated evenly even if the generation of heat by the pyrogen 5 only occurs partially, so that good vapour generation can still be achieved.

EXAMPLE

The pyrogen used in each of these embodiments preferably has the following composition:

| Iron oxide | 90 mesh | 46.4 wt% |
|---|---|---|
| Active carbon | 100 mesh | 20 wt% |
| Excelsior | | 17.2 wt% |
| Sodium chloride | | 4.1 wt% |
| Water | | 12.3 wt% |

**Claims**

1. A method of moxibustion in which heat is applied to the moxa by means of a pyrogen placed in thermal contact with the moxa and reactive with oxygen to generate heat.

2. A method as claimed in Claim 1 in which the pyrogen is packaged in an oxygen free form and is exposed to oxygen at the time of application of the moxa to the skin.

3. A method of moxibustion which comprises feeding air to a pyrogen consisting of a heat generating composition to cause it to develop heat by oxidation action, and arranging the heat so generated to heat moxa herb material placed in contact with the pyrogen such as to generate vapours of the ingredients of the moxa herb and to cause both these vapours and the heat to function on the surface of the skin.

4. A moxibustion device comprising a package body having a region permeable to air which in use will be applied to the skin, a moxa composition being located in the package body juxtaposed to the said permeable region and a pyrogen reactive with oxygen, located within the package body in thermal contact with the said moxa composition but within an oxygen impermeable envelope.

5. A moxibustion device in which a pyrogen, which will generate heat on contact with air, is packed in air free condition in the interior of a package body having air permeability to the side which in use will contact the skin, the package containing herb material in contact with the pyrogen.

6. A moxibustion device as claimed in Claim 4 or Claim 5 in which the pyrogen is contained in an impermeable envelope which is perforatable or is provided with holes covered by a removable seal.

7. A moxibustion device as claimed in Claim 4 or Claim 5 in which the pyrogen is contained in an air permeable envelope which is enclosed within an outer impermeable envelope either removable on use or provided with holes covered by a removable seal.

8. A moxibustion device as claimed in Claim 4, 5, 6 or 7 in which a separation member is located between the pyrogen and the moxa material.

9. A moxibustion device as claimed in Claim 4, 5, 6, 7 or 8 in which the moxa material is supported on a supporting member as a layer which is juxtaposed to the pyrogen.

10. A moxibustion device as claimed in Claim 9 in which the supporting member is black coloured.

11. A moxibustion device as claimed in Claim 10 in which supporting member is black coloured Kraft paper.

12. A moxibustion device as claimed in any one of Claims 9 to 11 in which venting holes are formed in the suporting member and are optionally covered by a seal removable from outside the device.

13. A moxibustion device consisting of a package body having a top and a bottom wall, and with air feeding holes in the top wall of the package body, the air feeding holes being sealed with a peelable sealing strip or board, the package body containing a pyrogen which generates heat on contact with oxygen, the pyrogen being supported on a separation board adhered to the edges of the package body, and a

supporting body, having venting holes and adhered to the separation board, with a space between the separating board and the supporting board, the space being filled with herb material.

14. A moxibustion device in which the package body has top and bottom walls and the edges of the top wall and bottom wall are adhered together and the space between filled with a pyrogen, which generates heat on exposure to oxygen, either one or both of the top and bottom walls being provided with air feeding holes, the edge of one surface of the package body being adhered to a supporting body of gas permeable material which is made by applying adhesive to the supporting body and adhering a peelable paper or strip thereto, and a herb supporting plate having herb material adhered to it inserted between the supporting body and the package body so that the herb material is located on the supporting body side.

15. A moxibustion method or device as claimed in any one of Claims 1 to 14 in which moxa is used as the herb material.

16. A moxibustion method or device as claimed in any one of Claims 1 to 14 in which moxa mixed with any one of ginger, garlic, Swertia japoneca, Angelica utilis, or a mixture thereof is used as the herb material.

## Patentansprüche

1. Moxibustionsverfahren bei dem der Moxa Wärme zugeführt wird mittels eines wärmeabgebenden Stoffes, der in thermischem Kontakt mit der Moxa angeordnet ist und zum Erzeugen von Wärme mit Sauerstoff reagiert.

2. Verfahren nach Anspruch 1, wobei der wärmeabgebende Stoff unter Ausschluß von Sauerstoff verpackt ist und zum Zeitpunkt der Anwendung der Moxa auf der Haut Sauerstoff ausgesetzt wird.

3. Moxibustionsverfahren mit folgenden Schritten: Zuführen von Luft an einen wärmeabgebenden Stoff, der eine wärmeerzeugende Mischung aufweist, wodurch dieser infolge eines Oxidationsvorgangs Wärme entwickelt, und Verwenden der so erzeugten Wärme zum Erwärmen des Moxa-Krautmaterials, das mit dem wärmeabgebenden Stoff in Kontakt angeordnet ist, so daß Dämpfe der Inhaltsstoffe des Moxa-Krauts erzeugt werden, und daß sowohl diese Dämpfe als auch die Wärme auf die Oberfläche der Haut einwirken.

4. Moxibustionsvorrichtung mit einem Paketkörper, der einen luftdurchlässigen Bereich hat, der bei der Verwendung auf die Haut aufgebracht wird, einer Moxa-Mischung, die in dem Paketkörper benachbart zu dem durchlässigen Bereich angeordnet ist, und einem mit Sauerstoff reagierenden, wärmeabgebenden Stoff, der innerhalb des Paketkörpers in thermischen Kontakt mit der Moxa-Mischung aber innerhalb einer sauerstoffundurchlässigen Hülle angeordnet ist.

5. Moxibustionsvorrichtung, wobei ein wärmeabgebender Stoff, der in Kontakt mit Luft Wärme erzeugt, unter Ausschluß von Luft im Innern eines Paketkörpers verpackt ist, der zu der Seite, die bei der Verwendung in Berührung mit der Haut steht, luftdurchlässig ist, wobei die Verpackung ein mit dem wärmeabgebenden Stoff in Berührung stehendes Krautmaterial enthält.

6. Moxibustionsvorrichtung nach Anspruch 4 oder 5, wobei der wärmeabgebende Stoff in einer undurchlässigen Hülle enthalten ist, die perforierbar ist oder Löcher aufweist, die mit einem entfernbaren Verschluß abgedeckt sind.

7. Moxibustionsvorrichtung nach Anspruch 4 oder 5, wobei der wärmeabgebende Stoff in einer luftdurchlässigen Hülle enthalten ist, die in einer äußeren, undurchlässigen Hülle eingeschlossen ist, die bei Verwendung entfernbar ist oder Löcher aufweist, die mit einem entfernbaren Verschluß abgedeckt sind.

8. Moxibustionsvorrichtung nach Anspruch 4, 5, 6 oder 7, wobei ein Trennelement zwischen dem wärmeabgebenden Stoff und dem Moxa-Material angeordnet ist.

9. Moxibustionsvorrichtung nach Anspruch 4, 5, 6, 7 oder 8, wobei das Moxa-Material auf einem Stützelement gelagert ist, das als Schicht benachbart zu dem wärmeabgebenden Stoff angeordnet ist.

10. Moxibustionsvorrichtung nach Anspruch 9, wobei die Farbe des Stützelements schwarz ist.

11. Moxibustionsvorrichtung nach Anspruch 10, wobei das Stützelement schwarzfarbiges Hartpapier ist.

12. Moxibustionsvorrichtung nach einem der Ansprüche 9 bis 11, wobei Belüftungslöcher in dem Stützelement ausgebildet sind, die gegebenenfalls mit einem von außerhalb der Vorrichtung entfernbaren Verschluß abgedeckt sind.

13. Moxibustionsvorrichtung mit einem Paketkörper, der eine Deck- und eine Bodenwand hat, und mit Luftzufuhrlöchern in der Deckwand des Paketkörpers, wobei die Luftzufuhrlöcher mit einem abziehbaren Verschlußstreifen oder -platte verschlossen sind, wobei der Paketkörper aufweist einen wärmeabgebenden Stoff, der bei Kontakt mit Sauerstoff Wärme erzeugt, wobei der wärmeabgebende Stoff auf einer Trennplatte, die an den Kanten des Paketkörpers angeklebt ist, gelagert ist und einem Stützkörper, der Belüftungslöcher hat und an der Trennplatte angeklebt ist, wobei ein Raum zwischen der Trennplatte und der Stützplatte vorhanden ist und dieser Raum mit Krautmaterial gefüllt ist.

14. Moxibustionsvorrichtung, wobei der Paketkörper eine Deck- und Bodenwand aufweist, und die Kanten der Deckwand und der Bodenwand miteinander verklebt sind, und der Raum dazwischen mit einem wärmeabgebenden Stoff gefüllt ist, der bei Kontakt mit Sauerstoff Wärme erzeugt, wobei die Deck- und/oder die Bodenwand Luftzufuhrlöcher aufweist, die Kante einer Fläche des Paketkörpers an einem Stützkörper aus gasdurchlässigem Material angeklebt ist, das hergestellt ist durch Aufbringen eines Klebers auf den Stützkörper und Daraufkleben eines abziehbaren Papiers oder Streifens, und wobei eine Kraut-Stützplatte mit einem darauf aufgeklebten Krautmaterial zwischen dem Stützkörper und dem Paketkörper so eingesetzt wird, daß das Krautmaterial auf der Seite des Stützkörpers angeordnet ist.

15. Moxibustionsverfahren oder -vorrichtung nach einem der Ansprüche 1 bis 14, wobei Moxa als Krautmaterial verwendet wird.

16. Moxibustionsverfahren oder -vorrichtung nach einem der Ansprüche 1 bis 14, wobei Moxa, das mit Ingwer, Knoblauch, japanischer Swertia und/oder Angelica utilits gemischt ist, als Krautmaterial verwendet wird.

**Revendications**

1. Procédé de moxibustion suivant lequel on applique de la chaleur au moxa à l'aide d'un pyrogène placé en contact thermique avec le moxa et capable de réagir avec l'oxygène pour engendrer de la chaleur.

2. Procédé selon la revendication 1, suivant lequel on conditionne le pyrogène sous une forme exempte d'oxygène, et on l'expose à l'oxygène au moment de l'application du moxa sur la peau.

3. Procédé de moxibustion qui comprend l'apport d'air à un pyrogène consistant en une composition thermogène, pour l'amenerà développer de la chaleur par action d'oxydation et la mesure consistant à faire en sorte que la chaleur ainsi engendrée chauffe le matériel herbacé à base de moxa, placé en contact avec le pyrogène, de façon à engendrer des vapeurs des ingrédients de l'herbe moxa et à amener à la fois ces vapeurs et la chaleur à agir sur la surface de la peau.

4. Dispositif de moxibustion comprenant un corps d'emballage présentant une région perméable à l'air, qui, à l'utilisation, sera appliquée sur la peau, une composition de moxa étant placée dans le corps d'emballage, en étant juxtaposée à ladite région perméable, et un pyrogène capable de réagir avec l'oxygène, placé à l'intérieur du corps d'emballage, en contact thermique avec ladite composition de moxa, mais à l'intérieur d'une enveloppe imperméable à l'oxygène.

5. Dispositif de moxibustion dans lequel un pyrogène, qui engendrera de la chaleur au contact de l'air, est conditionné à l'abri de l'air, à l'intérieur d'un corps d'emballage présentant une perméabilité à l'air sur le côté qui, à l'utilisation, sera en contact avec la peau, l'emballage contenant un matériel herbacé en contact avec le pyrogène.

6. Dispositif de moxibustion selon la revendication 4 ou la revendication 5, dans lequel le pyrogène est contenu dans une enveloppe imperméable, qui est perforable ou qui est dotée de trous recouverts par un élément de scellement détachable.

7. Dispositif de moxibustion selon la revendication 4 ou la revendication 5, dans lequel le pyrogène est contenu dans une enveloppe perméable à l'air qui est enfermée à l'intérieur d'une enveloppe imperméable externe, que est, soit détachable à l'utilisation, soit dotée de trous recouverts par un élément de scellement détachable.

8. Dispositif de moxibustion selon la revendication 4, 5, 6 ou 7, dans lequel un élément de séparation est placé entre le pyrogène et le matériel à base de moxa.

9. Dispositif de moxibustion selon la revendication 4, 5, 6, 7 ou 8, dans lequel le matériel à base de moxa est supporté par un élément de support, se présentant sous la forme d'une couche qui est juxtaposée au pyrogène.

10. Dispositif de moxibustion selon la revendication 9, dans lequel l'élément de support est coloré en noir.

11. Dispositif de moxibustion selon la revendication 10, dans lequel l'élément de support est un papier Kraft coloré en noir.

12. Dispositif de moxibustion selon l'une des revendications 9 à 11, dans lequel des trous de ventilation sont formés dans l'élément de support et sont facultativement recouverts par un élément de scellement détachable de l'extérieur du dispositif.

13. Dispositif de moxibustion constitué par un corps d'emballage présentant une paroi supérieure et une paroi inférieure, et avec des trous pour l'introduction de l'air dans la paroi supérieure du corps d'emballage, les trous pour l'introduction de l'air étant obturés de façon étanche par une bande ou plaque de scellement pelable, le corps d'emballage contenant un pyrogène qui engendre de la chaleur au contact de l'oxygène, le pyrogène étant porté par une plaque de séparation fixée aux bordures du corps d'emballage, et un corps de support, ayant des trous de ventilation et collé à la plaque de séparation, avec un espace entre la plaque de séparation et la plaque de support, l'espace étant rempli de matériel herbacé.

14. Dispositif de moxibustion dans lequel le corps d'emballage présente des parois supérieure et inférieure et les bordures de la paroi supérieure et de la paroi inférieure sont collées ensemble et l'espace intermédiaire est rempli d'un pyrogène, qui engendre de la chaleur lors de l'exposition à l'oxygène, l'une ou l'autre des parois inférieure et supérieure ou les deux étant dotées de trous pour l'introduction de l'air, la bordure de l'une des surfaces du corps d'emballage étant collée à un corps de support fait d'un matériau perméable aux gaz, que l'on réalise en appliquant un adhésif sur le corps de support et en collant un papier ou une bande pelable sur ledit adhésif, et une plaque de support d'herbe à laquelle adhère du matériel herbacé, étant insérée entre le corps de support et le corps d'emballage, de telle sorte que le matériel herbacé soit situé sur le côté du corps de support.

15. Procédé ou dispositif de moxibustion selon l'une des revendications 1 à 14, dans lequel le moxa est utilisé comme matériel herbacé.

16. Procédé ou dispositif de moxibustion selon l'une des revendications 1 à 14, dans lequel le moxa en mélange avec l'un quelconque parmi le gingembre, l'ail, Swertia japoneca, Angelica utilis, ou un de leurs mélanges, est utilisé comme matériel herbacé.

# FIG.1

# FIG.2

# FIG.3

EP 0 218 759 B1

## FIG.4

## FIG.5

## FIG.6

## FIG.7

## FIG.8